# EUROPEAN PATENT APPLICATION

(11) **EP 3 835 772 A1**
(43) Date of publication of application: **16.06.2021**
(21) Application number: 19847207.8
(22) Date of filing: 25.07.2019
(51) Int. Cl.: G01N 27/04, G01N 27/12

(54) **GAS DETECTOR**

(30) Priority: 10.08.2018 JP 2018151405
(71) Applicant: Figaro Engineering Inc., Minoo-shi, Osaka 562-0036 (JP); New Cosmos Electric Co., Ltd., Osaka-shi, Osaka 532-0036 (JP); University Public Corporation Osaka, Osaka-shi, Osaka 545-0051 (JP)
(72) Inventor: TAKEUCHI, Masato, Sakai-shi, Osaka 599-8531 (JP); FURUNO, Junpei, Sakai-shi, Osaka 599-8531 (JP); FUKUI, Kenta, Sakai-shi, Osaka 599-8531 (JP); IZAWA, Kuniyuki, Minoo-shi, Osaka 562-0036 (JP); SAI, Masakazu, Minoo-shi, Osaka 562-0036 (JP); MITSUHASHI, Hirokazu, Osaka-shi, Osaka 532-0036 (JP); TANIGUCHI, Takafumi, Osaka-shi, Osaka 532-0036 (JP)
(74) Representative: Kuhnen & Wacker Patent- und Rechtsanwaltsbüro PartG mbB
(86) International application number: PCT/JP2019/029239
(87) International publication number: WO 2020/031724

(57) **Abstract**

A gas detection unit is accommodated within a housing of a gas sensor and the outside atmosphere of the housing is introduced through the filter to the gas detection unit. The filter comprises an organic polymer gas-permeable filter removing siloxanes and an inorganic filter removing alcohols and passing gases to be detected.

## Description

### Field of the Invention

The present invention relates to gas detectors such as gas sensors and, in particular, to their filters.

### Background Art

Organic polymer gas-permeable membranes such as PTFE (poly-tetra-fluoro-ethylene) membranes have been used as filters of gas sensors (see Patent Document 1: JP2008-128687A) . Further, gas-selectively permeable membranes with high gas permeability and high gas selectivity have been developed (see Non-Patent Document 1: Energy-efficient polymeric gas separation membranes for a sustainable future: A review; Davide T. Sanders, et al. Polymer 54 (2013) 4719-4761). Both PTFE membranes and gas-selectively permeable membranes rapidly permeate hydrogen, methane, and other small molecular gases but slowly permeate toluene and other high molecular gases.

Gas sensors have the problem of siloxane poisoning. Since siloxanes have large molecular weights, organic polymer gas-permeable membranes are a good candidate for a siloxane filter. However, organic polymer gas-permeable membranes select molecules according to their molecular weights, they are not suitable for removing ethanol in the detection of methane, LPG (liquid petroleum gas), CO, and similar gases. As has been well known, ethanol is a typical daily miscellaneous gas and hinders the detection of desired gases. Typical other miscellaneous gases that hinder the detection of gases are acetone, iso-propanol, and methanol.

### Prior Document List

### Patent Documents

Patent Document 1: JP2008-128687A
Patent Document 2: WO2017-138190A

### Non-Patent Document

Non-Patent Document 1: Energy-efficient polymeric gas separation membranes for a sustainable future: A review; Davide T. Sanders, et al. Polymer 54 (2013) 4719-4761

### Summary of the Invention

### Problem to be Solved by the Invention

The object of the invention is to provide a new filter that removes both siloxanes and miscellaneous gases such as ethanol for gas detectors.

### Means for Solving the Problem

A gas detector according to the invention comprises a gas detection unit, and a filter introducing the surrounding atmosphere to the gas detection unit, and the filter comprises an organic polymer filter comprising a gas-permeable organic polymer membrane, removing siloxanes, and passing gases to be detected and an inorganic filter removing alcohols and passing the gases to be detected. Preferably, the gas detector is a gas sensor, and the gas sensor further comprises a housing accommodating the gas detection unit. Here, the filter is attached to the housing. In addition, the filter may be provided outside the housing. For example, a gas sensor without the filter may be accommodated in the downstream end, or the like, of a suction pipe, and, at the upstream position of the pipe, for example, at the tip end of the pipe, the filter according to the invention may be provided. The filter functions the same with this configuration to one attached to the housing.

According to the invention, the filter of the gas detector comprises or consists of an organic polymer filter that is gas-permeable and removes siloxanes and an inorganic filter that removes alcohols and passes gases to be detected or the desired gases. The organic polymer filter separates molecules according to their molecular weights or the like and prevents the permeation of siloxanes. The suitable materials for the organic polymer filter are, for example, fluoro-polymer resin membranes such as PTFE membranes, highly gas-permeable organic polymer synthetic resin membranes used as gas-selectively permeable membranes. The fluoro-polymer resin membranes include those with relatively low gas permeability such as PTFE and those with relatively high gas permeability such as Teflon AF (A registered trademark of E. I. Dupont), Hyflon AD (A registered trademark of Solvay), and Cytop (A registered trademark of Asahi Glass). Alternatively, the polymer organic membranes may be cellulose membranes, fucoidan membranes, chitosan membranes, and other polysaccharide membranes. These organic polymer membranes are gas-permeable and separate gases according to their molecular weights or both their molecular weights and the interaction between the membranes and the molecules. The species of organic polymer membranes are arbitrary. These membranes remove siloxanes having large molecular weights from the outside atmosphere and permeate methane, LPG, CO, and other desired gases and also alcohols such as ethanol. While LPG permeates more slowly the organic polymer membranes than methane, it is enough if a high concentration LPG is detected, and the organic polymer filter does not hinder the detection of LPG.

The inorganic filter comprises or consists of active charcoal, silica-gel, zeolite, meso-porous silica, aluminum silicate, or another inorganic adsorbent, or alternatively, an oxidation catalyst such as noble metal, or metal oxide. Preferably, the above oxidation catalyst comprises a support consisting or the aforementioned inorganic adsorbent and oxidation catalyst such as noble metal catalyst and metal oxide catalyst supported on the support, or alternatively, a polymer fiber support other than adsorbents (for example, PTFE fiber) and oxidation catalyst such as noble metal catalyst and metal oxide catalyst supported on the fiber. Particularly preferably, the oxidation catalyst comprises noble metal catalyst and the aforementioned inorganic adsorbent or the polymer fiber. The inorganic adsorbent adsorbs ethanol, methanol, iso-propanol, other alcohols, acetone, and similar substances. The oxidation catalyst removes these miscellaneous gases by oxidation. When an inorganic adsorbent supporting the oxidation catalyst is used, both the adsorption of miscellaneous gases and the oxidation of them proceed. The forms of the inorganic filter are arbitrary; for example, granular active charcoal and sheet-like active charcoal fibers are usable. These inorganic adsorbents, except for those having small pore diameters such as MS5A (Molecular Sieve 5A), adsorb siloxanes.

Either of the organic polymer filter and the inorganic filter may be allocated towards the outside atmosphere. Preferably, the organic polymer filter works as a pre-filter and the inorganic filter works as a post-filter such that the inorganic filter may treat the atmosphere permeated the organic polymer filter. Particularly preferably, the inorganic filter including the oxidation catalyst treats the atmosphere which has permeated the organic polymer filter. When siloxanes are already removed at an upstream position than the oxidation catalyst, the poisoning of the oxidation catalyst as well as the poisoning of the gas detection unit is prevented. Most preferably, two organic polymer filters are provided at the outside of the inorganic filter (atmosphere side) and at the inside of the inorganic filter (gas detection unit side). Then, the gas detection unit is securely protected from poisoning.

When the organic polymer filter and the inorganic filter are integrated, the organic polymer filter may be overlayed on a sheet-like inorganic filter or may be formed as a membrane on a support comprising the sheet-like inorganic filter. In the latter case, the fibers or the particles of the inorganic filter are integrated with the organic polymer membrane.

According to the invention, the organic polymer membrane removes siloxanes in the outside atmospheres, and the inorganic filter removes alcohols. Thus, the gas detector, or the gas sensor can detect the desired gases such as methane, LPG, and CO without the poisoning by siloxanes and without false detection by alcohols.

Preferably, the organic polymer membrane includes an acidic group or a basic group. Particularly preferably, the organic polymer membrane comprises carboxy-methyl-cellulose including carboxyl group, cellulose sulfate, fucoidan, other acidic polysaccharides, or chitosan including amino group.

The organic polymer membranes prevent the permeation of siloxanes, and the heat of solution or the heat of adsorption of siloxanes in the organic polymer membranes is large. Therefore, siloxanes are expected to stay for a long while in the membrane. However, when siloxanes are accumulated in the membrane, the siloxanes may permeate the membrane and poison the gas detection unit.

The inventors have confirmed meso-porous silica with the introduction of sulfo group makes siloxanes polymerized (Patent Document 2). The speculated polymerization mechanism is the hydrolysis of siloxanes by the sulfo group. While siloxane molecules adsorbed in the pores of meso-porous silica can desorb into the surrounding gas phase due to the equilibrium between adsorption and desorption, the siloxane molecules diffused in organic polymer membranes are expected to be more restricted in their movement. Therefore, carboxyl group and phosphoric acid group, weaker in their acidity than sulfo group, can fix and polymerize siloxanes. Further, since the polymerization of siloxanes is expected to proceed with hydrolysis, basic groups such as amino group, basic hydroxyl group, and so on, are expected to polymerize siloxanes.

Nafion (A registered trademark of E. I. Dupont) has a framework of fluoro-polymer resin and sulfo group and is easily available as a solution. Therefore, gas-selectively permeable membranes and Nafion can be easily blended, and, for example, desired membranes can be formed by mixing a solution of gas-selectively permeable membrane materials and the Nafion solution and then by forming the membranes. Alternatively, the gas-selectively permeable membrane materials may be blended with organic polymer anion ion conductor with a basic hydroxyl group.

Some of polysaccharides include carboxyl group such as carboxy-methyl-cellulose, sulfo group such as fucoidan and cellulose sulfate, and amino group such as chitosan. In addition, polysaccharide membranes can be easily formed in general and the resultant membranes are gas-permeable. Therefore, by these membranes, siloxanes are chemically fixed in the membranes by the polymerization and so on.

### Brief Description of the Drawings

Fig. 1 is a cross-sectional view of a gas sensor according to the embodiment.
Fig. 2 is a cross-sectional view of a laminated membrane according to the embodiment.
Fig. 3 is a plan view of a chip according to the embodiment.
Fig. 4 indicates a driving pattern for the gas sensor according to the embodiment.
Fig. 5 is a characteristic view revealing the durability of the embodiment and that of a comparative example, against siloxane (D5 x 100ppm).
Fig. 6 is a characteristic view revealing the sensitivities of the embodiment and a comparative example to 200ppm ethanol.

### Features for Carrying out the Invention

The best embodiment for carrying out the invention will be described.

### Embodiment

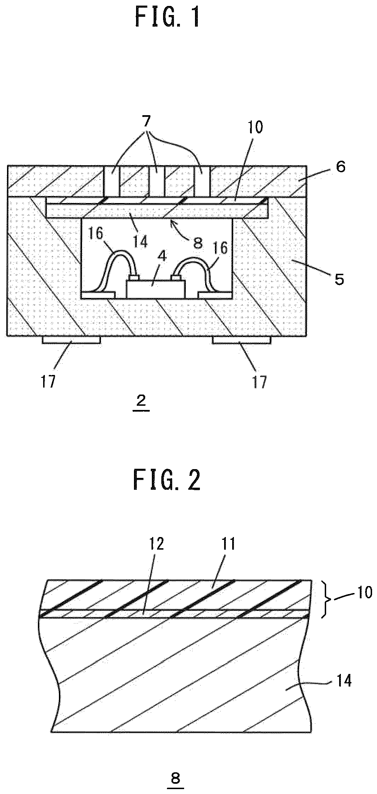

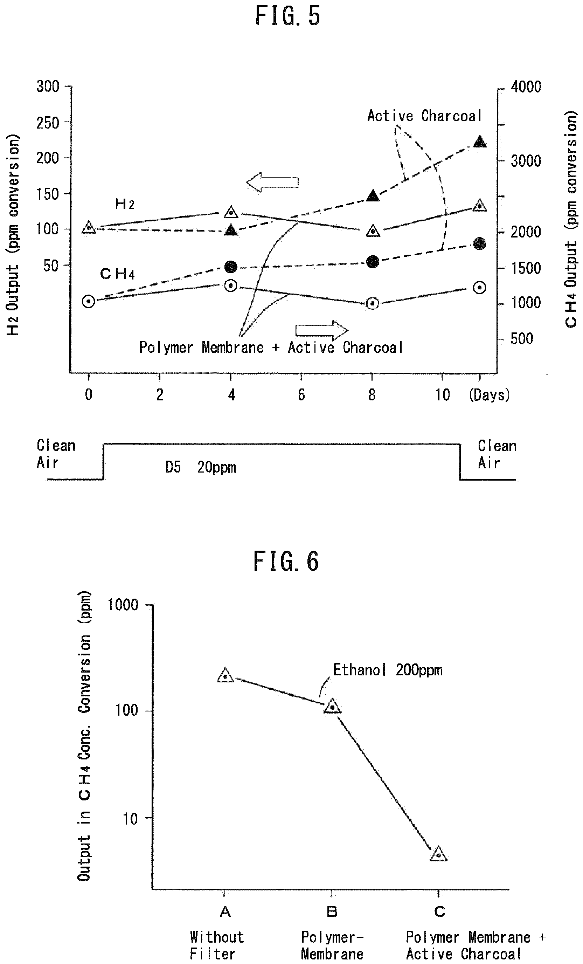

Figs. 1 to 4 indicate a gas sensor 2 according to the embodiment, and Figs. 5 and 6 indicate test results. The gas sensor 2 is, for example, provided with a Si chip 4, which is an example of a gas detection unit. The Si chip 4 is accommodated in a housing 5, such as a ceramic housing, and is fixed in the housing 5, for example, by die bonding. The opening of the housing 5 is covered by a ceramic lid 6 so that atmosphere outside the housing 5 is supplied through plural openings 7 to a filter 8. On the inside surface of the lid 6 (the surface towards the Si chip 4), the membranous filter 8 is attached. The filter 8 comprises the two layers of an organic polymer filter 10 and an active charcoal filter 14; the organic polymer filter 10 is positioned towards the outside of the housing 5 and the active charcoal filter 14 is towards the Si chip 4. The active charcoal filter 14 is an example of the inorganic filter and may be replaced by an oxidation catalyst filter, or the like. it is preferable to allocate the organic polymer filters 10 at both sides of the inorganic filter. Further, the inorganic filter may comprise laminated distinct inorganic filters. The species of gas detection unit and structure of the housing are arbitrary. In addition, the pads of the Si chip 4 are connected to terminals 17 on the housing 5 via leads 16.

Fig. 2 indicates the construction of the filter 8. The organic polymer filter 10 comprises a porous support membrane 11 and a gas-permeable organic membrane 12 overlayed on the support membrane, but the support membrane 11 may be omitted. The support membrane 11 is a synthetic resin membrane or a polysaccharide membrane having continuous pores and has a thickness of, for example, 1 micro-meter to 100 micro-meter. The gas-permeable organic polymer membrane 12 has a thickness of, for example, 0.1 micro-meter to 5 micro-meter. The active charcoal filter 14 in the embodiment comprises an active charcoal fiber sheet having a thickness of about 1 mm, but granular active charcoal, silica gel, meso-porous silica, zeolite, aluminum-silicate may be used. The shape and the material for the inorganic filter are arbitrary. Further, in place of the active charcoal filter 14, an oxidation catalyst filter may be usable. The oxidation catalyst filter comprises the above adsorbents and an oxidation catalyst such as a noble metal supported on the adsorbents or polymer fibers and an oxidation catalyst such as a noble metal supported on the fibers.

The gas-permeable organic polymer membrane 12 comprises a polysaccharide membrane or a gas-selective permeable membrane made from a synthetic polymer. Since long-chain molecules in polysaccharide membranes tend to be regularly ordered, continuous micro-pores are easily generated. These micro-pores work as the gas diffusion path and the sizes of the micro-pores determine the sizes of molecules that can permeate the membrane. Namely, siloxanes having larger molecular diameters can not permeate but hydrogen, methane, LPG, CO, ethanol, and so on, can permeate the membrane. Gas-selective permeable membranes comprising synthetic polymers are generally highly gas-permeable, their gas permeability depends upon the molecular sizes. They permeate hydrogen, methane, CO, LPG, ethanol, and so on, but do not permeate siloxanes. The polymer membrane 12 may be formed by casting; spin-coating; spray-coating, roll-coating; and so on.

When an acidic group, such as carboxyl group, sulfo group, or phosphoric acid group, or a basic group, such as amino group or basic hydroxyl group is introduced into the gas-permeable organic polymer membrane 12 (hereinafter, simply "polymer membrane 12"), these groups bind with the - (O-Si-O) - portion in the siloxane molecules. When the siloxane concentration in the membrane increases, the siloxane molecules are hydrolyzed at the - (O-Si-O) - portion and are polymerized such that they are completely fixed in the membrane. Therefore, even when exposed to high concentration siloxanes for a long while, the siloxanes do not permeate the organic polymer filter 12.

Preferable polymer membrane 12 comprises a polysaccharide, such as carboxy-methyl-cellulose; cellulose-sulfate; fucoidan; and chitosan, and an acidic group, such as carboxyl group (carboxy-methyl-cellulose) and sulfo group (cellulose sulfate and fucoidan), or a basic group such as amino group (chitosan). Instead of using the polysaccharide membrane, an acidic or a basic group may be introduced into synthetic resin gas-selectively permeable membranes. For example, a protonic-conductive polymer such as Nafion or a hydroxyl ion-conductive polymer may be blended with gas-selectively permeable membranes comprising fluoropolymers.

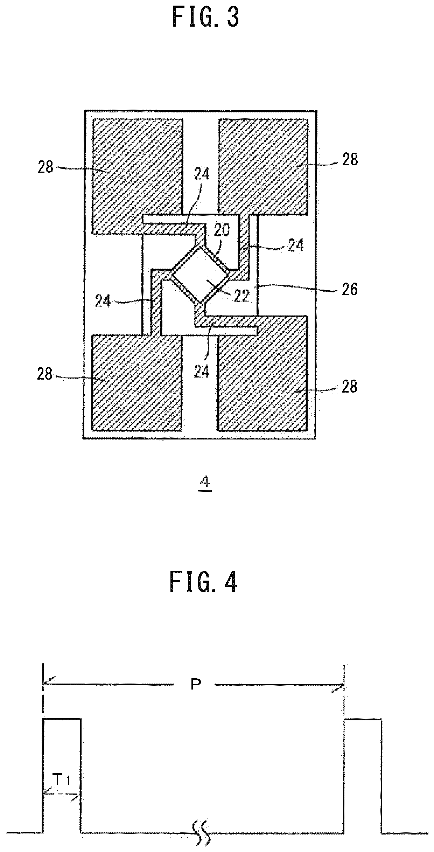

Fig. 3 indicates the Si chip 4; the Si chip 4 is provided with a micro-hotplate 20 with electrodes and a heater, on a cavity 26. The hotplate 20 is supported by beams 24 and has a metal oxide semiconductor 22 on it. Indicated by 28 are pads.

Other gas detection units than the Si chip 4 are usable, and other gas detection materials than the metal oxide semiconductors are usable. For example, a contact combustion catalyst is usable as the gas detection material and, in this case, is supported on the hotplate 20 or by a heater coil not shown in the drawings. In addition, the metal oxide semiconductor 22 may be supported by other means than the hotplate 20. Further, electrochemical gas sensors that have a liquid or solid electrolyte and detection and counter electrodes connected to the electrolyte or further have a reference electrode connected to the electrolyte are usable as the gas detection unit.

Fig. 4 indicates the operational pattern of the gas sensor 2. The gas sensor 2 is operated with a period P, is heated to an operational temperature of 250 degree Celsius to 450 degree Celsius (200 degree Celsius in the embodiment) for a period T1 for each period P, and gases are detected based upon the resistance of the metal oxide semiconductor when heated.

Fig. 5 shows the results of a durability test to siloxane (exposure for ten days in 20ppm D5). The tested gas sensors are shown in Figs. 1 to 3; the embodiment shown by solid lines was provided with the polymer membrane (carboxy-methyl-cellulose) and sheet-like active charcoal, and the comparative example was provided with only the sheet-like active charcoal. The outputs in 100ppm hydrogen were measured and they were converted into hydrogen concentrations by the initial dependence of the outputs on hydrogen concentrations. The shifts of the concentrations from 100ppm indicate the degree of being poisoned, and, when poisoned by siloxanes, the outputs converted into concentrations generally increase. Similarly, the outputs in 1000ppm methane were measured for the estimation of siloxane durability. The organic polymer filter 10 reduces the influence of siloxane. As a remark, as is obvious from the above, the organic polymer filter 10 prevents the oxidation catalyst filter from being poisoned when the oxidation catalyst filter is used in place of the active charcoal filter 14.

Fig. 6 indicates the methane concentrations that give the same outputs to 200ppm ethanol; A indicates a comparative example without any filter, B a comparative example with only a polymer membrane (carboxy-methyl-cellulose), and C the embodiment with the polymer membrane (carboxy-methyl-cellulose) and sheet-like active charcoal. It is indicated that ethanol can not be removed by the polymer membrane but removed by the active charcoal.

### List of Symbols

- 2: gas sensor
- 4: Si chip (gas detection unit)
- 5: housing
- 6: lid
- 7: opening
- 8: filter
- 10: organic polymer membrane
- 11: support membrane
- 12: gas-permeable organic membrane
- 14: active charcoal filter (inorganic filter)
- 16: lead
- 17: terminal
- 20: micro-hotplate
- 22: metal oxide semiconductor
- 24: beam
- 26: cavity
- 28: pad

## Claims

1. A gas detector comprising: a gas detection unit; and a filter introducing surrounding atmosphere to the gas detection unit,
wherein the filter comprises an organic polymer filter comprising a gas-permeable organic polymer membrane, removing siloxanes, and passing gases to be detected and an inorganic filter removing alcohols and passing the gases to be detected.

2. The gas detector according to claim 1,
wherein the gas detector is a gas sensor, and
wherein the gas sensor further comprises a housing accommodating the gas detection unit and wherein said filter is attached to the housing.

3. The gas detector according to claim 2,
wherein said organic polymer filter is allocated towards outside atmosphere of the housing and said inorganic filter is allocated towards the gas detection unit both in the housing.

4. The gas detector according to one of claims 1 to 3,
wherein said inorganic filter includes oxidation catalyst.

5. The gas detector according to one of claims 1 to 4,
wherein said gas-permeable organic polymer membrane includes an acidic group or a basic group.

6. The gas detector according to claim 5,
wherein said gas-permeable organic polymer membrane comprises one of the group consisting of carboxy-methyl-cellulose including carboxyl group, cellulose sulfate, fucoidan, chitosan including amino group.
